# EUROPEAN PATENT APPLICATION

(11) **EP 4 831 209 A1**
(43) Date of publication of application: **28.10.2026**
(21) Application number: 24906413.0
(22) Date of filing: 18.12.2024
(51) Int. Cl.: C07K 19/00, A61K 39/00, A61K 38/20, A61P 35/00, C12N 15/62, C12N 5/10, C12N 15/867

(54) **ENGINEERED TIL EXPRESSING MEMBRANE-BOUND IL-15 FUSION PROTEIN AND USE THEREOF**

(30) Priority: 19.12.2023 CN 202311752324
(71) Applicant: Shanghai Chuangyinsai Biotechnology Co., Ltd., Shanghai 201210 (CN)
(72) Inventor: SUN, Zhen, Qingdao, Shandong 266108 (CN); XU, Aotian, Qingdao, Shandong 266108 (CN); YU, Zhongjie, Qingdao, Shandong 266108 (CN); ZHAO, Yi, Qingdao, Shandong 266108 (CN); GAO, Qing, Qingdao, Shandong 266108 (CN)
(74) Representative: dompatent
(86) International application number: PCT/CN2024/140355
(87) International publication number: WO 2025/130934

(57) **Abstract**

The present invention relates to a membrane-bound IL-15 fusion protein expressed on a TIL cell. Specifically, in the present invention, a hypoxia response element (HRE) is used to induce the expression of the membrane-bound IL-15 (mIL-15) fusion protein, so that a TIL cell population expressing the fusion protein persistently maintain a memory stem cell phenotype for a long time. thereby enhancing the ani-tumor activity thereof. During the construction of the fusion protein, a CD86 transmembrane region and an intracellular domain are used, thereby further improving the proportion of stem cells in the TIL cells. During molecule construction, a truncated form of hEGFRt is used as a molecular switch to further enhance the safety. The stemness and persistence of the TIL cells expressing the fusion protein of the present invention are significantly enhanced, and persistent immune surveillance and therapeutic efficacy can be provided.

## Description

### TECHNICAL FIELD

The present invention relates to the field of genetic engineering and cell therapy technology, and in particular to engineered TIL expressing membrane-bound IL-15 fusion protein and use thereof.

### BACKGROUND

The incidence of cancer is increasing year by year and showing a trend of younger patients. Among all types of cancer, solid tumors account for approximately 90%. Due to the complexity, variability, and strong heterogeneity of solid tumors, they are extremely difficult to cure. In recent years, following surgery, radiotherapy, chemotherapy, and immunotherapy, cell therapy has gradually demonstrated its powerful therapeutic effects in the treatment of various cancers. With the rapid development of human tumor treatment technology, adoptive cell therapy (ACT) has gradually become one of the treatment options for patients with advanced tumors. Chimeric antigen receptor T cell (CAR-T) therapy, engineered T cell receptor T cell (TCR-T) therapy, tumor infiltrating lymphocyte (TIL) therapy, etc. all fall within the category of ACT. Compared with CAR-T and TCR-T, TIL cell drugs have demonstrated significant efficacy in the treatment of solid tumors and have become one of the most promising technical directions for conquering solid tumors.

The specific killer lymphocytes exist in the tumor microenvironment are called tumor-infiltrating lymphocytes (TIL). TIL therapy is an adoptive cell therapy that utilizes the patient's own immune system to treat tumors. It has been successfully used in patients with metastatic melanoma and other solid tumors. TILs are composed of T cells with multiple TCR clones, enabling them not only to directly recognize tumor-associated antigens, but also target tumor-specific neoantigens, making them more effective in dealing with tumor heterogeneity. TIL expresses chemokine receptors after being stimulated by tumor antigens *in vivo*, which makes it easier for TIL to be localized in tumor tissue after being reinfused. In addition, TIL are derived from the patient's own body, which means that this method has low toxicity.

However, TIL therapy obviously has its limitations. The usual method for preparing TIL is to obtain a single-cell suspension after mechanical treatment or enzymatic digestion of tumor tissue, followed by culture with high concentrations of IL2 to selectively expand T lymphocytes, reaching billions of TILs within 2-4 weeks and then reinfused into the patient. Reinfusion therapy is accompanied by high-dose IL2 infusion to support the growth and activity of TILs injected into the patient. However, high-dose IL2 can lead to dose-limiting toxicity, requiring intensive monitoring and care; low-dose IL2 may be beneficial to peripheral tolerance and the production of regulatory T cells, thereby inhibiting the anti-tumor response of TILs; in addition, the IL2 signaling pathway drives the proliferation of effector T cells, promoting terminal differentiation and exhaustion. The above disadvantages greatly limit the clinical application of TIL therapy.

To reduce the toxicity associated with high-dose IL2 and improve the *in vivo* survival and function of traditional TIL therapy, there is an urgent need to develop engineered TIL cells with improved stemness, persistence, and anti-tumor activity.

### SUMMARY OF THE INVENTION

The purpose of the present invention is to provide an engineered TIL with improved stemness, persistence and anti-tumor activity.

In the first aspect of the present invention, it provides a fusion polypeptide comprising the following elements:
(i) interleukin 15 (IL-15) or a functionally active fragment thereof;
(ii) a transmembrane domain; and
(iii) an intracellular domain;
wherein, the intracellular domain is a CD86 intracellular domain.

In another preferred embodiment, the CD86 intracellular domain comprises a full-length intracellular domain, or an active fragment thereof.

In another preferred embodiment, the IL-15 comprises a full-length IL-15, a mature form of IL-15, or an active fragment thereof.

In another preferred embodiment, the IL-15 comprises wild type and mutant type.

In another preferred embodiment, the amino acid sequence of IL-15 is set forth in SEQ ID NO: 1.

In another preferred embodiment, the fusion polypeptide further comprises a hinge region.

In another preferred embodiment, the fusion polypeptide further optionally comprises a signal peptide and/or a linker.

In another preferred embodiment, the linker is located between the element (i) and the element (ii), between the element (ii) and the element (iii), between the element (i) and the hinge region, or between the hinge region and the element (ii).

In another preferred embodiment, the linker comprises a flexible linker or a rigid linker.

In another preferred embodiment, the amino acid sequence of the linker is set forth in SEQ ID NO: 5.

In another preferred embodiment, the structural formula of the fusion polypeptide is as shown in formula (I):

X-IL15-L-H-TM-Cyto (I)

wherein,
"-" is independently absent or a linking peptide;
X is absent or a signal peptide;
IL15 is the interleukin 15 element;
L is absent or a linker;
H is a hinge region;
TM is a transmembrane domain;
Cyto is an intracellular domain, wherein the intracellular domain is a CD86 intracellular domain.

In another preferred embodiment, the amino acid sequence of the CD86 intracellular domain is set forth in SEQ ID NO: 2.

In another preferred embodiment, the transmembrane domain is a CD86 transmembrane domain, preferably, the amino acid sequence of the CD86 transmembrane domain is set forth in SEQ ID NO: 3.

In another preferred embodiment, the hinge region is a CD86 hinge region, preferably, the amino acid sequence of the CD86 hinge region is set forth in SEQ ID NO:4.

In another preferred embodiment, the amino acid sequence of the signal peptide is set forth in SEQ ID NO: 6.

In another preferred embodiment, the sequence of the fusion protein is set forth in SEQ ID NO: 7.

In the second aspect of the present invention, it provides a polynucleotide, which encodes the fusion polypeptide of the present invention.

In the third aspect of the present invention, it provides a nucleic acid construct, comprising a first expression cassette, wherein the first expression cassette comprises a hypoxia-responsive promoter and the polynucleotide of the second aspect of the present invention.

In another preferred embodiment, the hypoxia-responsive promoter is a promoter comprising n hypoxia response elements (HREs), ie, a promoter comprising n×HRE elements, wherein n is an integer selected from 1 to 20.

In another preferred embodiment, n is an integer selected from 2 to 9; more preferably, n is an integer selected from 2 to 4; most preferably, n is 3.

In another preferred embodiment, the hypoxia-responsive promoter is selected from the group consisting of: a TK-mini promoter comprising n×HRE elements, a CMV-mini promoter comprising n×HRE elements, and an IL2-mini promoter comprising n×HRE elements.

In another preferred embodiment, the nucleic acid construct further comprises a sequence of a molecular switch element, and the molecular switch element is selected from the group consisting of: hEGFRt, BCMA, and CD20.

In another preferred embodiment, the molecular switch element is hEGFRt.

In another preferred embodiment, the molecular switch element is linked to the first expression cassette via a cleavable linker peptide sequence.

In another preferred embodiment, the cleavable linker peptide is a self-cleaving 2A peptide, preferably a T2A peptide.

In another preferred embodiment, the nucleic acid construct comprises a second expression cassette, wherein the second expression cassette comprises a second promoter and a molecular switch element coding sequence.

In another preferred embodiment, the second promoter is an SFFV promoter.

In the fourth aspect of the present invention, it provides a vector comprising the polynucleotide of the second aspect of the present invention.

In another preferred embodiment, the vector comprises a plasmid or a viral vector.

In another preferred embodiment, the viral vector comprises: a lentiviral vector, an adenoviral vector, and a yellow fever virus vector.

In another preferred embodiment, the vector is a plasmid.

In the fifth aspect of the present invention, it provides an immune cell, which comprises the fusion polypeptide of the present invention, the polynucleotide of the second aspect of the present invention, the nucleic acid construct of the third aspect of the present invention, and/or the vector of the fourth aspect of the present invention.

In another preferred embodiment, the immune cell is selected from the group consisting of: a tumor infiltrating cell (TIL), T cell, and NK cell.

In another preferred embodiment, the cell is a tumor infiltrating cell (TIL).

In the sixth aspect of the present invention, it provides a composition, which comprises the fusion polypeptide of the present invention, the polynucleotide of the second aspect of the present invention, the nucleic acid construct of the third aspect of the present invention, the vector of the fourth aspect of the present invention, and/or the cell of the fifth aspect of the present invention, and a pharmaceutically acceptable carrier.

In the seventh aspect of the present invention, it provides a kit, which comprises the fusion polypeptide of the present invention, the polynucleotide of the second aspect of the present invention, the nucleic acid construct of the third aspect of the present invention, the vector of the fourth aspect of the present invention, the cell of the fifth aspect of the present invention, and/or the composition of the sixth aspect of the present invention.

In the eighth aspect of the present invention, it provides a use of the fusion polypeptide of the present invention, the polynucleotide of the second aspect of the present invention, the nucleic acid construct of the third aspect of the present invention, the vector of the fourth aspect of the present invention, the cell of the fifth aspect of the present invention, the composition of the sixth aspect of the present invention, and/or the kit of the seventh aspect of the present invention in the preparation of a medicament for preventing, alleviating and/or treating a tumor.

In another preferred embodiment, the tumor comprises a solid tumor, a hematological tumor, or a combination thereof.

In another preferred embodiment, the tumor is selected from the group consisting of: lung cancer, cervical cancer, gastrointestinal cancer, pancreatic cancer, glioblastoma, ovarian cancer, bladder cancer, liver cancer, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

In the ninth aspect of the present invention, it provides a method for treating a disease, comprising administering the immune cell of the fifth aspect of the present invention or the composition of the sixth aspect of the present invention to a subject.

In another preferred embodiment, the disease is a tumor.

In another preferred embodiment, the tumor comprises a solid tumor, a hematological tumor, or a combination thereof.

In another preferred embodiment, the tumor is selected from the group consisting of: lung cancer, cervical cancer, gastrointestinal cancer, pancreatic cancer, glioblastoma, ovarian cancer, bladder cancer, liver cancer, breast cancer, colon cancer, rectal cancer, colorectal cancer, endometrial or uterine cancer, salivary gland cancer, kidney cancer, prostate cancer, vulvar cancer, thyroid cancer, liver cancer, anal cancer, penile cancer, testicular cancer, esophageal cancer, bile duct tumor, and head and neck cancer.

It should be understood that, within the scope of the present invention, the above-described technical features of the present invention and the technical features specifically described below (such as in the examples) can be combined with each other to form new or preferred technical solutions. Due to space limitations, they will not be described in detail here.

### DESCRIPTION OF THE DRAWINGS

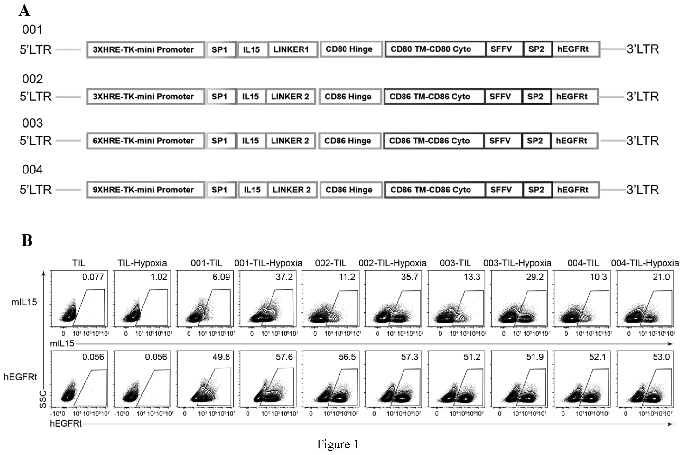

Figure 1 shows lentiviral infection of TIL cells. Figure 1, panel A shows a schematic diagram of the structures of 001, 002, 003, and 004 plasmids; Figure 1, panel B shows the results of flow cytometry analysis of the proportions of mIL-15 and hEGFRt after TIL cells were infected with lentiviruses with different plasmid structures.

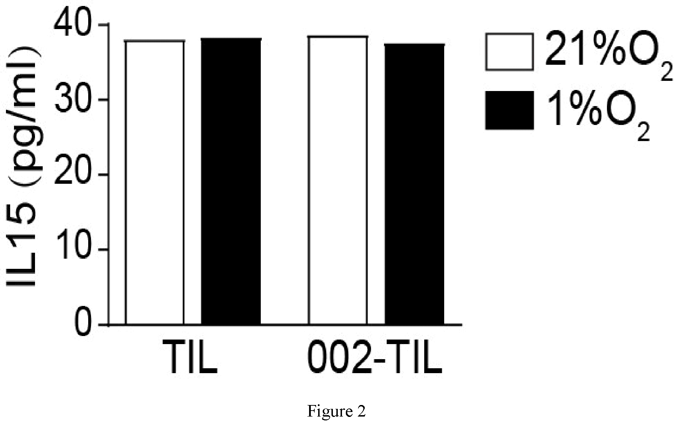

Figure 2 shows the shedding of mIL-15 detected by ELISA. Compared with the control group and the non-hypoxia group, no significant difference in IL-15 secretion was observed after hypoxia treatment, indicating that the membrane-bound IL-15 did not undergo shedding.

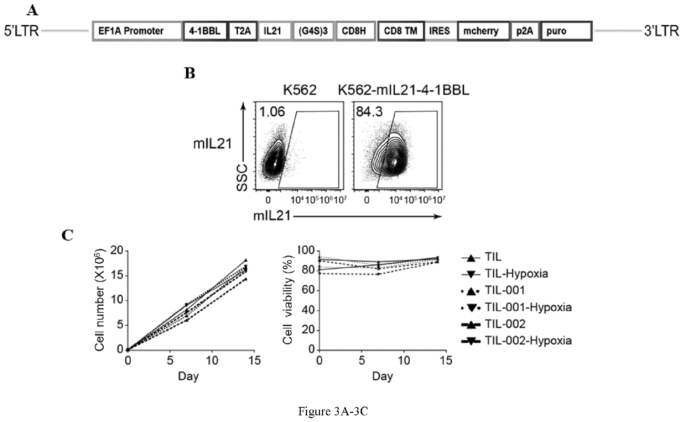

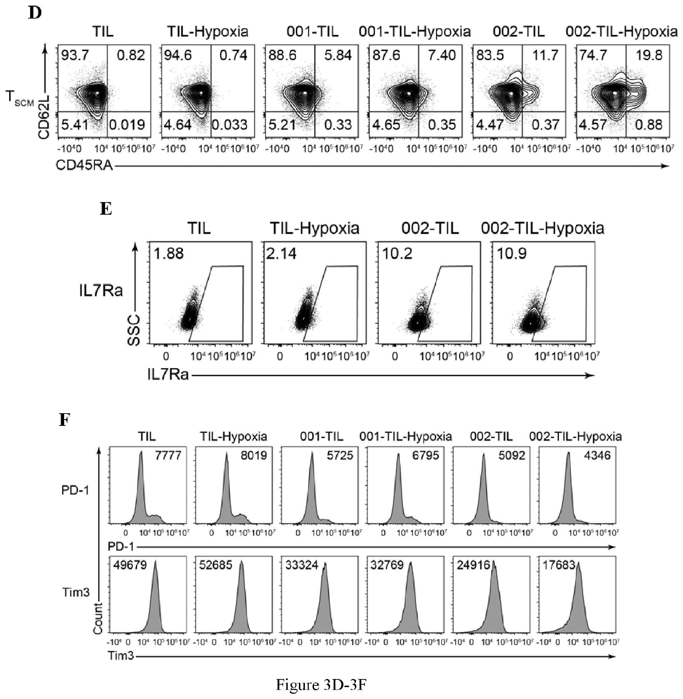

Figure 3 shows the flow cytometry results of T_{SCM} and T cell exhaustion. Figure 3, panel A shows a schematic diagram of the structure of feeder cell K562-mIL21-4-1BBL; Figure 3, panel B shows the flow cytometry detection of mIL21 in K562 and K562-mIL21-4-1BBL cells; Figure 3, panel C shows the cell counting and viability records of TILs infected with lentiviruses carrying different plasmid constructs after co-cultured with feeder cells K562-mIL21-4-1BBL for 7 and 14 days; Figure 3, panel D shows the flow cytometry detection of T_{SCM} in TILs infected with lentiviruses carrying different plasmid constructs after co-cultured with feeder cells K562-mIL21-4-1BBL for 18 days; Figure 3, panel E shows the flow cytometry detection of IL7Rα in TILs infected with lentiviruses carrying different plasmid constructs after co-cultured with feeder cells K562-mIL21-4-1BBL for 18 days; Figure 3, panel F shows the flow cytometry detection of CD8+ T cell exhaustion in TILs infected with lentiviruses carrying different plasmid constructs after co-cultured with feeder cells K562-mIL21-4-1BBL for 18 days.

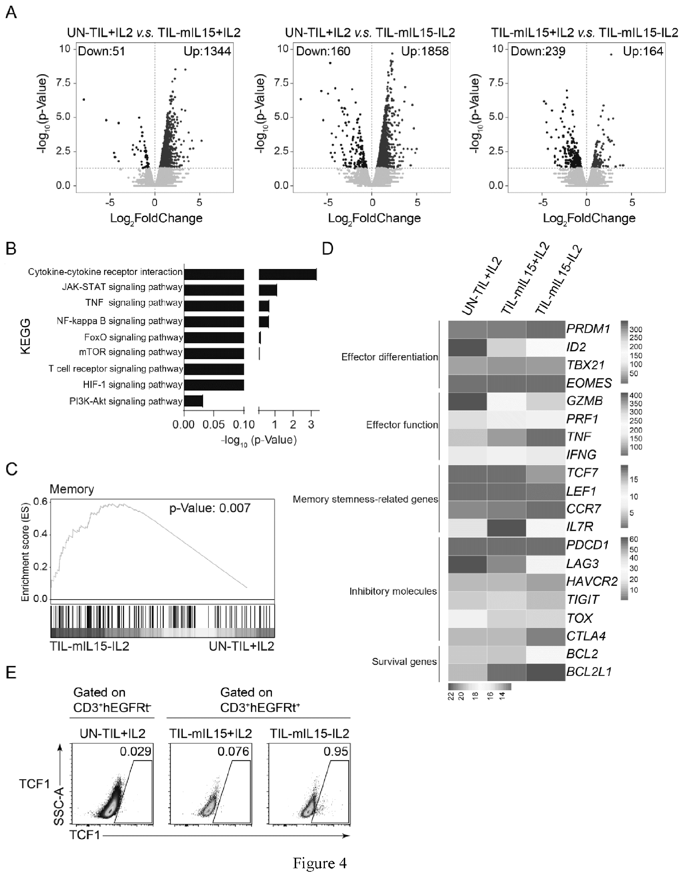

Figure 4 shows the RNA-Seq and flow cytometry analysis of UN-TIL+IL2, TIL-mIL-15+IL2, and TIL-mIL-15-IL2 cells after 14 days of expansion on the K562-mIL21-4-1BBL cell culture platform. Figure 4, panel A shows a volcano plot showing upregulated (red) or downregulated (blue) genes in TIL-mIL-15-IL2 cells compared with UN-TIL+IL2 and TIL-mIL-15+IL2 cells. Figure 4, panel B shows KEGG pathway analysis showing the expression of differentially expressed genes in TIL-mIL-15-IL2 cells relative to UN-TIL+IL2 cells. Figure 4, panel C shows GSEA analysis showing the gene signatures in TIL-mIL-15-IL2 cells relative to UN-TIL+IL2 cells. Figure 4, panel D shows a heat map showing the expression of effector differentiation, effector function, memory stemness-related genes, inhibitory molecules, and survival genes. Figure 4, panel E shows the expression of TCF1 in CD3+hEGFRt- and CD3+hEGFRt+ cells from UN-TIL+IL2, TIL-mIL-15+IL2, and TIL-mIL-15-IL2 cells (-IL2: without IL2 addition; +IL2: with IL2 addition).

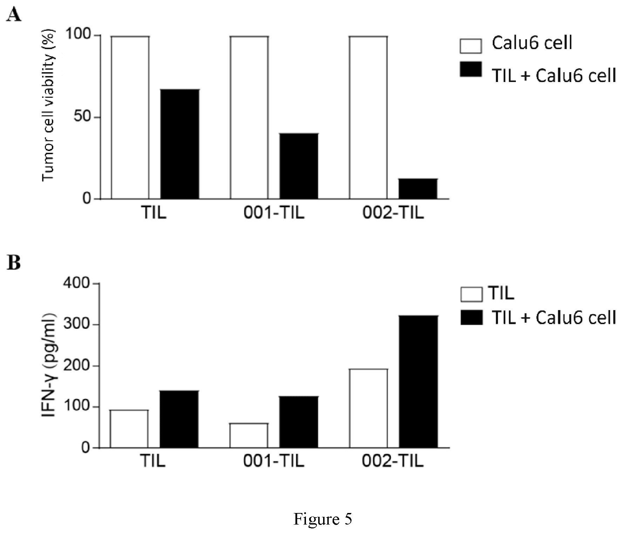

Figure 5 shows the anti-tumor activity of TIL cells overexpressing mIL-15. Figure 5, panel A shows that TIL, 001-TIL, and 002-TIL cells were co-cultured with tumor cells Calu6 for 24 hours, and tumor cell activity was detected by CCK8 assay. Figure 5, panel B shows that TIL, 001-TIL, and 002-TIL cells were co-cultured with Calu6 tumor cells for 24 hours, and IFN-γ secretion was detected by ELISA.

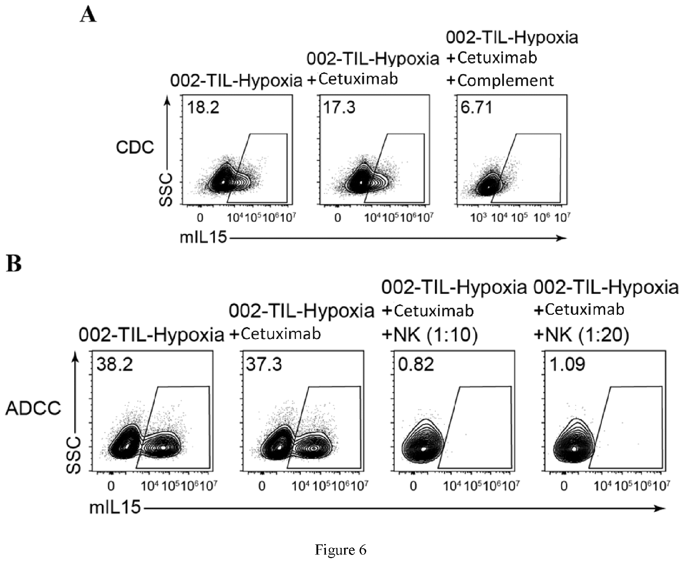

Figure 6 shows the flow cytometry results of mIL-15. Figure 6, panel A shows the flow cytometry detection of mIL-15+ cells in 002-TIL cells after treatment with mAb, complement, and mAb for 4 hours; Figure 6, panel B shows the flow cytometry detection of mIL-15+ cells in 002-TIL cells after treatment with mAb, NK cells, and mAb for 24 hours.

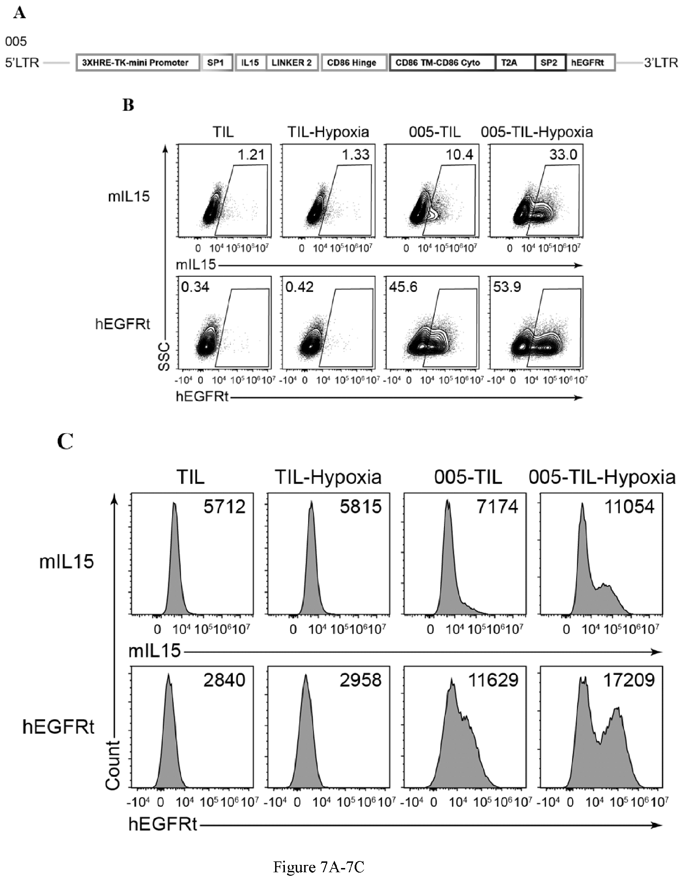

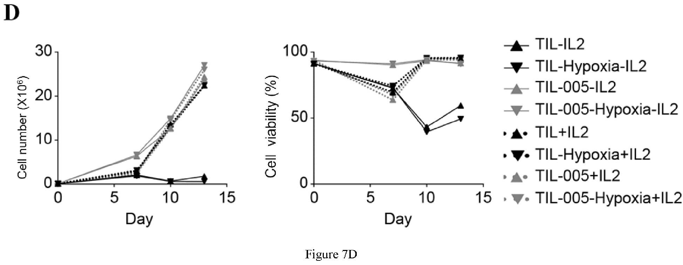

Figure 7 shows the results of the detection of TIL cells infected with 005 lentivirus. Figure 7, panel A shows a schematic diagram of the 005 plasmid structure; Figure 7, panel B shows the proportion of mIL-15 and hEGFRt detected by flow cytometry after 005 lentivirus infection of TIL; Figure 7, panel C shows the gMFI of mIL-15 and hEGFRt detected by flow cytometry after 005 lentivirus infection of TIL; Figure 7, panel D shows the *in vitro* proliferation and survival of TIL and 005-TIL cells with and without the addition of IL2 (-IL2: without IL2 addition; +IL2: with IL2 addition).

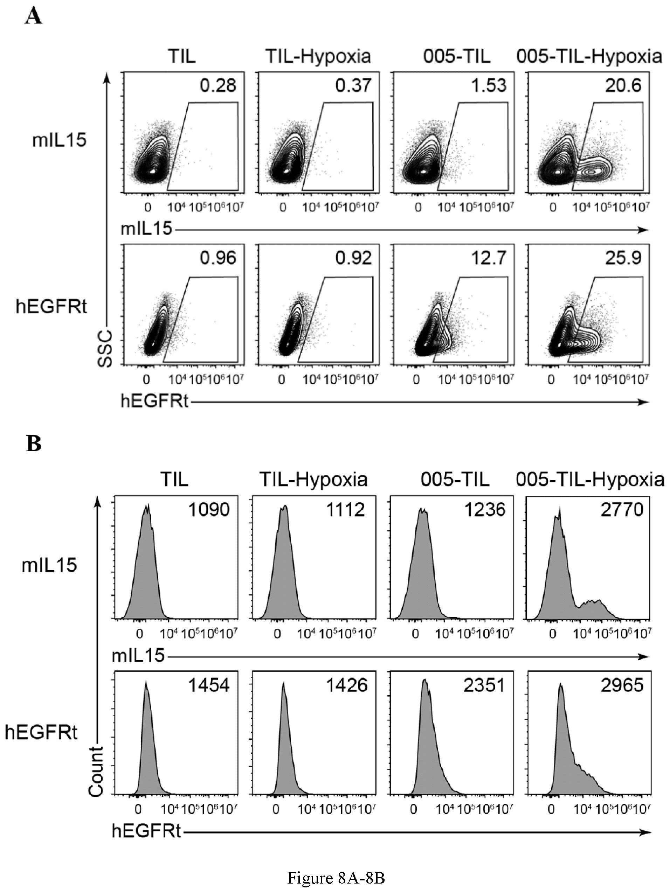

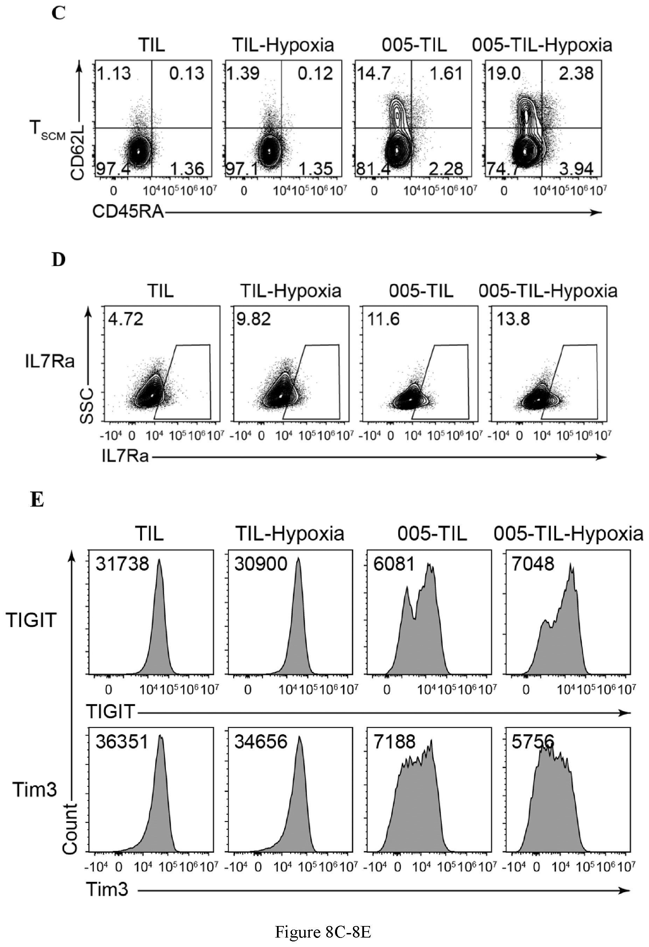

Figure 8 shows the results of TIL cells from cervical cancer infected with the 005 lentivirus. Figure 8, panel A shows the proportions of mIL-15+ and hEGFRt+ cells in TILs infected with the 005 lentivirus as detected by flow cytometry; Figure 8, panel B shows the gMFI of mIL-15 and hEGFRt in TILs infected with the 005 lentivirus; Figure 8, panel C shows flow cytometry detection of T_{SCM} in TILs infected with the 055 lentivirus after co-cultured with feeder cells K562-mIL21-4-1BBL for 18 days; Figure 8, panel D shows flow cytometry detection of IL7Rα in TILs infected with the 055 lentivirus after co-cultured with feeder cells K562-mIL21-4-1BBL for 18 days; Figure 8, panel E shows the expression of exhaustion markers on CD8+ T cells after infection with the 055 lentivirus and co-cultured with feeder cells K562-mIL21-4-1BBL for 18 days, as detected by flow cytometry.

### DETAILED DESCRIPTION

Through extensive and in-depth research, the inventors have for the first time provided a membrane-bound IL-15 fusion protein with a specific structure. The fusion protein (the fusion protein of the present invention) comprises an IL-15 element, a CD86 transmembrane region and an intracellular domain. The fusion protein of the present invention unexpectedly significantly increases the proportion of stem cells in TIL cells and significantly enhances their anti-tumor activity. In addition, the present invention further uses the hypoxia response element HRE to induce the expression of the fusion protein of the present invention, thereby allowing the engineered TIL cells to express the fusion protein of the present invention in a hypoxic environment and maintain the long-term persistence of the memory stem cell phenotype. The present invention has been accomplished on this basis.

Specifically, the present inventors, in modifying TIL cells, preferably employ a hypoxia response element (HRE) to induce expression of the fusion protein of the present invention, and use truncated hEGFRt as a molecular switch in the construct to further enhance safety. The engineered TIL cells of the present invention exhibit significantly improved stemness and persistence, providing sustained immune surveillance and therapeutic capacity.

### Fusion polypeptide of the present invention

As used herein, the terms "the fusion polypeptide of the present invention", "the fusion protein of the present invention", "the membrane-bound IL-15 of the present invention" and "the mIL-15 of the present invention" are used interchangeably and all refer to the fusion protein of the present invention comprising IL-15, a transmembrane domain and an intracellular domain.

Interleukin IL-15 is a pro-survival cytokine that maintains homeostasis of long-lived CD8+ memory T cells, inhibits activation-induced cell death (AICD), enhances anti-tumor activity *in vivo*, and reverses T cell anergy. Monomeric IL-15 is a small, unstable protein with a short serum half-life, requiring supraphysiological administration to achieve an *in vivo* response. In the present invention, IL-15 is linked to a transmembrane domain and an intracellular domain to obtain membrane-bound IL-15, which has the function of maintaining the long-term persistence of the memory stem cell phenotype.

In the present invention, the preferred intracellular domain is the CD86 intracellular domain. The use of the CD86 intracellular domain can further increase the proportion of stem T cells and reduce the expression of T cell exhaustion molecules. The preferred transmembrane domain of the present invention is the CD86 transmembrane domain.

The fusion protein of the present invention may further comprise a hinge region, a signal peptide and/or a linker. The preferred structural formula of the fusion protein of the present invention is shown in formula (I):

X-IL15-L-H-TM-Cyto (I)

wherein,
"-" is independently absent or a linking peptide;
X is absent or a signal peptide;
IL15 is interleukin 15;
L is absent or a linker;
H is a hinge region; the preferred hinge region of the present invention is the CD86 hinge region;
TM is a transmembrane domain; the preferred transmembrane domain of the present invention is the CD86 transmembrane domain;
Cyto is an intracellular domain, and the preferred intracellular domain of the present invention is the CD86 intracellular domain.

Wherein, the preferred amino acid sequence of IL-15 is as follows:

NWVNVISDLKKIEDLIQSMHIDATLYTESDVHPSCKVTAMKCFLLELQVISLESGDA
SIHDTVENLIILANNSLSSNGNVTESGCKECEELEEKNIKEFLQSFVHIVQMFINTS (SEQ
ID NO: 1).

Preferably, the amino acid sequence of the CD86 intracellular domain is as follows:

KWKKKKRPRNSYKCGTNTMEREESEQTKKREKIHIPERSDEAQRVFKSSKTSSCDK
SDTCF (SEQ ID NO: 2)

Preferably, the amino acid sequence of the CD86 transmembrane domain is WITAVLPTVIICVMVFCLILW (SEQ ID NO: 3).

Preferably, the amino acid sequence of the CD86 hinge region is LEDPQPPPDHIP (SEQ ID NO: 4).

Preferably, the amino acid sequence of the linker is SGGGSGGGGSGGGGSGGGGSGGGS (SEQ ID NO: 5).

Preferably, the amino acid sequence of the signal peptide is MDWTWILFLVAAATRVHS (SEQ ID NO: 6).

Preferably, the amino acid sequence of the fusion protein of the present invention is as follows:

MDWTWILFLVAAATRVHSNWVNVISDLKKIEDLIQSMHIDATLYTESDVHPSCKVTA
MKCFLLELQVISLESGDASIHDTVENLIILANNSLSSNGNVTESGCKECEELEEKNIKEFLQ
SFVHIVQMFINTSSGGGSGGGGSGGGGSGGGGSGGGSLEDPQPPPDHIPWITAVLPTVIIC
VMVFCLILWKWKKKKRPRNSYKCGTNTMEREESEQTKKREKIHIPERSDEAQRVFKSSK
TSSCDKSDTCF (SEQ ID NO: 7).

### Nucleic acid construct of the present invention

As used herein, the nucleic acid construct of the present invention can express the fusion protein of the present invention. The nucleic acid construct of the present invention comprises a first expression cassette encoding the fusion protein of the present invention, wherein the first expression cassette has a hypoxia-responsive promoter.

The hypoxia-responsive promoter of the present invention is a promoter comprising n hypoxia response elements HRE, i.e., an n×HRE promoter. Hypoxia is a hallmark of solid tumors, and this unique environmental signal can be used for targeted cancer therapy. When constructing a nucleic acid molecule expressing the fusion protein of the present invention, the present invention uses a hypoxia-responsive promoter to express the fusion protein of the present invention in hypoxic environment. There is no particular limitation on the type of promoter used to construct the hypoxia-responsive promoter of the present invention. Preferably, the present invention uses a TK mini promoter comprising n×HRE.

Preferably, the present invention uses the 3×HRE-TK-mini promoter, the sequence of which is set forth in SEQ ID NO: 8.

In one embodiment, the present invention uses a 6×HRE-TK-mini promoter, the sequence of which is set forth in SEQ ID NO: 9.

In one embodiment, the present invention uses the 9×HRE-TK-mini promoter, the sequence of which is set forth in SEQ ID NO: 10.

In one embodiment, the nucleic acid construct of the present invention further comprises a molecular switch element (also known as an immune brake element). When a cell expressing the nucleic acid construct presents a safety risk, the molecular switch element can be used as a target to eliminate the risky cell using a corresponding drug.

In one embodiment, the molecular switch element of the present invention can be linked to the first expression cassette via a cleavable linker peptide; preferably, the cleavable linker peptide is a self-cleaving peptide; preferably, the cleavable connecting peptide is T2A.

In one embodiment, the molecular switch element of the present invention is located in a second expression cassette, and expression of the molecular switch element is controlled by a second promoter. In one embodiment, the second promoter is an SFFV promoter, which is a constitutive promoter that can stably and efficiently drive expression of the molecular switch element. In one embodiment, the second expression cassette comprises a second signal peptide.

In one embodiment, the molecular switch or immune brake element of the present invention is hEGFRt. If cells comprising the nucleic acid construct of the present invention present a safety risk, the anti-hEGFRt therapeutic monoclonal antibody cetuximab can be injected to eliminate the target cells through ADCC and CDC, further improving safety. Flow cytometry of hEGFRt molecules can also be used to indicate the positive rate of cells with target gene integration.

In a preferred embodiment, a hypoxia-responsive mIL-15 nucleic acid construct having a molecular switch element is constructed, which comprises the following sequences in sequence: n×HRE-TK-mini promoter sequence, a first signal peptide sequence, an IL-15 sequence, a linker sequence, a CD86 hinge region sequence, a CD86 transmembrane region-intracellular domain sequence, an SFFV promoter sequence, a second signal peptide sequence, and a hEGFRt sequence.

Preferably, n in the n×HRE-TK-mini promoter is an integer selected from 2 to 9; more preferably, n is an integer selected from 2 to 4; most preferably, n is 3.

### The vector of the present invention

In the present invention, the term "vector" generally refers to a nucleic acid molecule capable of transporting another nucleic acid linked to it. One type of vector is a "plasmid," which refers to a circular double-stranded DNA loop into which other DNA segments can be ligated. Another type of vector is a viral vector, in which other DNA segments can be ligated to a viral genome. Certain vectors are capable of autonomous replication in the host cells into which they are introduced (e.g., bacterial vectors and episomal mammalian vectors with bacterial replication origins). Other vectors (e.g., non- episomal mammalian vectors) can be integrated into the genome of the host cell when introduced into the host cell, thereby replicating together with the host genome, such as naked RNA polynucleotides that cannot autonomously replicate, naked DNA polynucleotides, polynucleotides composed of DNA and RNA in the same chain, poly-lysine-coupled DNA or RNA, peptide-coupled DNA or RNA, liposome-coupled DNA, etc. In addition, certain vectors can direct the expression of genes effectively ligated to them. This type of vector is referred to as a "recombinant expression vector" (or simply "expression vector") in the present invention. Generally speaking, the expression vector used in recombinant DNA technology is typically in the form of a plasmid. In this specification, "plasmid" and "vector" are used interchangeably, as plasmid is the most commonly used form of vector.

As used herein, "the vector of the present invention" refers to a vector comprising the nucleic acid construct of the present invention. Preferably, the vector of the present invention is a plasmid.

### Engineered TILs of the present invention

As used herein, the terms "the engineered TILs of the present invention" or "the TILs of the present invention" are used interchangeably and refer to TILs that can express the fusion protein of the present invention and/or TILs comprising the vector of the present invention.

The engineered TILs of the present invention express membrane-bound IL-15 (mIL-15), which reduces T cell exhaustion by promoting the formation of T_{SCM}. T cell subsets corresponding to a less differentiated state offer the potential for superior clinical utility. T memory stem cells (T_{SCM}) are the least differentiated memory T cell subset identified to date, possessing the highest self-renewal capacity and therapeutic potential. The increased proportion of T_{SCM} in the TILs of the present invention enables long-term survival of the TIL cell population.

### Pharmaceutical composition of the present invention

The pharmaceutical composition of the present invention can comprise the fusion protein of the present invention or the immune effector cell of the present invention (such as the engineered TIL of the present invention) and one or more pharmaceutically acceptable carriers, diluents, excipients and adjuvants. These compositions can be suitable for use in the treatment of therapeutic indications as described herein.

The composition can be a liquid solution, suspension, emulsion, sustained release formulation or powder, and can be formulated with a pharmaceutically acceptable carrier. The composition can be formulated into a suppository using conventional adhesives and carriers such as triglycerides. "Pharmaceutically acceptable carrier" refers to a carrier matrix or vehicle that does not interfere with the effectiveness of the biological activity of the active ingredient and is non-toxic to the host or subject.

The fusion protein or immune effector cell can be delivered together with a pharmaceutically acceptable vehicle. In one embodiment, the vehicle can enhance stability and/or delivery properties. Vehicles are such as artificial membrane vesicles (including liposomes, nonionic surfactant vesicles (noisome), nano-microliposome, etc.), microparticles or microcapsules, or colloidal preparations comprising pharmaceutically acceptable polymers.

The pharmaceutical composition comprising one or more fusion proteins or immune effector cells can be prepared into sterile injectable aqueous or oily suspensions according to methods known in the art and using appropriate one or more dispersing agents or wetting agents and/or suspending agents. The sterile injectable preparation can be a sterile injectable solution or suspension in a non-toxic parent acceptable diluent or solvent.

In the present invention, the term "adjuvant" generally refers to any substance that assists or regulates the effects of drugs, including but not limited to immunological adjuvants, which enhance or diversify the immune response to antigens.

In the present invention, the term "subject" can be a mammal in need of treatment, such as a human or veterinary patient (e.g., a rodent, such as a mouse or rat, cat, dog, cow, horse, sheep, goat, or other livestock). In some embodiments, a "subject" can be a clinical patient, a clinical trial volunteer, an experimental animal, or the like. The subject may be suspected of having a disease characterized by cell proliferation or have a risk of developing a disease characterized by cell proliferation, be diagnosed with a disease characterized by cell proliferation, or be a control subject confirmed not to have a disease characterized by cell proliferation. As described herein, diagnostic methods for diseases characterized by cell proliferation and the clinical division of such diagnosis are known to those skilled in the art.

The pharmaceutical compositions of the present invention can be used to treat tumors. In the present invention, the term "tumor" or "tumor cell" generally refers to or describes a physiological condition in mammals that is generally characterized by unregulated cell growth. Examples of tumors comprise, but are not limited to, carcinomas, lymphomas, blastomas (including medulloblastoma and retinoblastoma), sarcomas (including liposarcoma and synovial cell sarcoma), neuroendocrine tumors (including carcinoid tumors, gastrinomas and pancreatic islet cell carcinomas), mesotheliomas, schwannomas (including acoustic neuromas), meningiomas, adenocarcinomas and melanomas. "Tumor cells" can further comprise "solid tumors", which refer to tumors selected from the group consisting of: gastrointestinal cancer, pancreatic cancer, glioblastoma, cervical cancer, ovarian cancer, bladder cancer, hepatoma, breast cancer, colon cancer, rectal cancer, colorectal cancer, endometrial or uterine cancer, salivary gland cancer, kidney cancer, prostate cancer, vulvar cancer, thyroid cancer, hepatic carcinoma, anal cancer, penile cancer, testicular cancer, esophageal cancer, bile duct tumors, and head and neck cancer.

### The main advantages of the present invention include:

(1) The present invention constructs for the first time a membrane-bound interleukin-15 fusion protein comprising the CD86 intracellular domain. The stemness, persistence and anti-tumor activity of TILs expressing this fusion protein are significantly improved.
(2) When constructing the nucleic acid construct for expressing the fusion protein, the present invention uses a hypoxia-responsive promoter, i.e., a promoter comprising an HRE element, which can induce the expression of the fusion protein of the present invention under the hypoxic environment that is characteristic of solid tumors.
(3) The present invention adds an immune molecular switch, such as hEGFRt, when constructing a nucleic acid construct expressing a fusion protein, thereby avoiding the potential toxicity of sustained expression of IL-15 and improving the safety of clinical treatment.

The present invention will be further described below in conjunction with specific examples. It should be understood that these examples are intended to illustrate the present invention only and are not intended to limit the scope of the present invention. The experimental methods in the following examples, for which specific conditions are not specified, are generally performed under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

The sequence information involved in the examples is shown in Table A.

**Table A nucleotide sequences of the elements**

| Element name | Nucleotide sequence | SEQ ID NO: |
|---|---|---|
| 3×HRE-TK -mini promoter | TGTCACGTCCTGCACGACTCTAGTTGTCACGTCCTGCACGACTC TAGTTGTCACGTCCTGCACGACGCTAGCTCGAGATCCGGCCCCG CCCAGCGTCTTGTCATTGGCGAATTCGAACACGCAGATGCAGTC GGGGCGGCGCGGTCCGAGGTCCACTTCGCATATTAAGGTGACG CGTGTGGCCTCGAACACCGAGCGACCCTGCAGCGACCCGCTTA A | 8 |
| 6×HRE-TK -mini promoter | TGTCACGTCCTGCACGACTCTAGTTGTCACGTCCTGCACGACTC TAGTTGTCACGTCCTGCACGACGCTAGCTACCGAGCTCTGTCAC GTCCTGCACGACTCTAGTTGTCACGTCCTGCACGACTCTAGTTG TCACGTCCTGCACGACGCTAGCTCGAGATCCGGCCCCGCCCAG CGTCTTGTCATTGGCGAATTCGAACACGCAGATGCAGTCGGGGC GGCGCGGTCCGAGGTCCACTTCGCATATTAAGGTGACGCGTGTG GCCTCGAACACCGAGCGACCCTGCAGCGACCCGCTTAA | 9 |
| 9×HRE-TK -mini promoter | TGTCACGTCCTGCACGACTCTAGTTGTCACGTCCTGCACGACTC TAGTTGTCACGTCCTGCACGACGCTAGCACCCGGGAGGTACCG AGCTCTGTCACGTCCTGCACGACTCTAGTTGTCACGTCCTGCAC GACTCTAGTTGTCACGTCCTGCACGACGCTAGCTACCGAGCTCT GTCACGTCCTGCACGACTCTAGTTGTCACGTCCTGCACGACTCT AGTTGTCACGTCCTGCACGACGCTAGCTCGAGATCCGGCCCCGC CCAGCGTCTTGTCATTGGCGAATTCGAACACGCAGATGCAGTCG GGGCGGCGCGGTCCGAGGTCCACTTCGCATATTAAGGTGACGC GTGTGGCCTCGAACACCGAGCGACCCTGCAGCGACCCGCTTAA | 10 |
| Signal peptide SP1 | ATGGATTGGACTTGGATTTTGTTTCTGGTAGCAGCCGCAACTCG GGTGCATTCC | 11 |
| IL-15 | AACTGGGTGAATGTAATAAGTGATTTGAAAAAAATTGAAGATCT TATTCAATCTATGCATATTGATGCTACTTTATATACGGAAAGTGAT GTTCACCCCAGTTGCAAAGTAACAGCAATGAAGTGCTTTCTCTT GGAGTTACAAGTTATTTCACTTGAGTCCGGAGATGCAAGTATTC ATGATACAGTAGAAAATCTGATCATCCTAGCAAACAACAGTTTG TCTTCTAATGGGAATGTAACAGAATCTGGATGCAAAGAATGTGA GGAACTGGAGGAAAAAAATATTAAAGAATTTTTGCAGAGTTTTG TACATATTGTCCAAATGTTCATCAACACTTCT | 12 |
| Linker LINKER2 | TCAGGGGGCGGAAGTGGGGGCGGGGGATCTGGTGGGGGAGGG AGCGGCGGCGGCGGCAGCGGAGGCGGATCC | 13 |
| CD86 hinge region | CTCGAAGACCCACAACCTCCCCCAGACCATATACCT | 14 |
| CD86 transmembr ane domain and intracellular domain | TGGATCACCGCCGTACTGCCGACTGTGATTATATGCGTTATGGTC TTCTGCCTGATCCTTTGGAAATGGAAAAAGAAGAAGAGACCCC GGAACTCTTACAAGTGTGGTACAAACACCATGGAGAGGGAGGA ATCAGAGCAAACCAAGAAGCGCGAAAAGATTCACATCCCAGAG AGAAGTGACGAAGCTCAGCGCGTCTTTAAGAGTTCTAAGACCA GCAGTTGTGACAAATCCGATACATGTTTC | 15 |
| SFFV promoter | GTAACGCCATTTTGCAAGGCATGGAAAAATACCAAACCAAGAAT AGAGAAGTTCAGATCAAGGGCGGGTACATGAAAATAGCTAACG TTGGGCCAAACAGGATATCTGCGGTGAGCAGTTTCGGCCCCGGC CCGGGGCCAAGAACAGATGGTCACCGCAGTTTCGGCCCCGGCC CGAGGCCAAGAACAGATGGTCCCCAGATATGGCCCAACCCTCA GCAGTTTCTTAAGACCCATCAGATGTTTCCAGGCTCCCCCAAGG ACCTGAAATGACCCTGCGCCTTATTTGAATTAACCAATCAGCCT GCTTCTCGCTTCTGTTCGCGCGCTTCTGCTTCCCGAGCTCTATAA AAGAGCTCACAACCCCTCACTCGGCGCGCCAGTCCTCCGACAG ACTGAGTCGCCCGGG | 16 |
| Signal peptide SP2 | ATGCTTCTCCTGGTGACAAGCCTTCTGCTCTGTGAGTTACCACA CCCAGCATTCCTCCTGATCCCA | 17 |
| hEGFRt | CGCAAAGTGTGTAACGGAATAGGTATTGGTGAATTTAAAGACTC ACTCTCCATAAATGCTACGAATATTAAACACTTCAAAAACTGCA CCTCCATCAGTGGCGATCTCCACATCCTGCCGGTGGCATTTAGG GGTGACTCCTTCACACATACTCCTCCTCTGGACCCACAGGAACT GGATATTCTGAAAACCGTAAAGGAAATCACAGGGTTTTTGCTGA TTCAGGCTTGGCCTGAAAACAGGACGGACCTCCATGCCTTTGA GAACCTAGAAATCATACGCGGCAGGACCAAGCAACATGGTCAG TTTTCTCTTGCAGTCGTCAGCCTGAACATAACATCCTTGGGATTA CGCTCCCTCAAGGAGATAAGTGATGGAGATGTGATAATTTCAGG AAACAAAAATTTGTGCTATGCAAATACAATAAACTGGAAAAAAC TGTTTGGGACCTCCGGTCAGAAAACCAAAATTATAAGCAACAG AGGTGAAAACAGCTGCAAGGCCACAGGCCAGGTCTGCCATGCC TTGTGCTCCCCCGAGGGCTGCTGGGGCCCGGAGCCCAGGGACT GCGTCTCTTGCCGGAATGTCAGCCGAGGCAGGGAATGCGTGGA CAAGTGCAACCTTCTGGAGGGTGAGCCAAGGGAGTTTGTGGAG AACTCTGAGTGCATACAGTGCCACCCAGAGTGCCTGCCTCAGG CCATGAACATCACCTGCACAGGACGGGGACCAGACAACTGTAT CCAGTGTGCCCACTACATTGACGGCCCCCACTGCGTCAAGACCT GCCCGGCAGGAGTCATGGGAGAAAACAACACCCTGGTCTGGAA GTACGCAGACGCCGGCCATGTGTGCCACCTGTGCCATCCAAACT GCACCTACGGATGCACTGGGCCAGGTCTTGAAGGCTGTCCAAC GAATGGGCCTAAGATCCCGTCCATCGCCACTGGGATGGTGGGGG CCCTCCTCTTGCTGCTGGTGGTGGCCCTGGGGATCGGCCTCTTC ATG | 18 |
| T2A peptide | GAGGGCAGAGGAAGTCTTCTAACATGCGGTGACGTGGAGGAGA ATCCCGGCCCT | 19 |

### Example 1: Lentiviral infection of TILs with the gene of interest

### 1. Plasmid construction

Based on the hypoxic microenvironment characteristics of solid tumors, four constructs were designed and generated using 3×HRE, 6×HRE, and 9×HRE elements for inducible expression. The constructs were named 001, 002, 003, and 004, and were subjected to whole-gene synthesis. As shown in Figure 1, panel A, the nucleotide sequence of IL-15 in each construct is set forth in SEQ ID NO: 12. Construct 001 served as a control, employing the CD80 transmembrane domain and CD80 intracellular domain, with the 3×HRE-TK-mini promoter. Constructs 002, 003, and 004 employed the CD86 transmembrane domain and CD86 intracellular domain, with the 3×HRE-TK-mini promoter, 6×HRE-TK-mini promoter, and 9×HRE-TK-mini promoter, respectively. All four constructs contained the second promoter SFFV, the second signal peptide SP2, and the molecular switch element hEGFRt. The plasmid construction methods for the four constructs were performed as follows:
(1) The CDS sequences of the relevant genes were downloaded from NCBI, and partial gene sequences were codon-optimized to enhance expression. These optimization strategies included, but were not limited to: human codon usage preference, moderate GC content, stable mRNA secondary structure, removal of repetitive sequences, cryptic splice sites, and unwanted restriction enzyme sites, while avoiding depletion of the cellular tRNA pool.
(2) The optimized sequences were subjected to whole-gene synthesis by GenScript and were cloned into the lentiviral shuttle vector pLV-EF1a-c-MYC-IRES-EGFP (Wuhan Miaoling), replacing the sequence from the EF1a promoter to EGFP with the target sequence. The newly constructed vector was named pLV-HRE-mIL-15.

### 2. Lentivirus preparation

HEK-293T cells were transfected with the pLV-HRE-mIL-15 containing the mIL-15 gene obtained in step 1, along with the psPAX2 and pMD2.G vector plasmids. The transfection mix was prepared as follows: (1) Tube A: 500 µl Opti-MEM + 10 µg of the main plasmid + 5 µg pMD2.G + 5 µg psPAX2 + 40 µl P3000; (2) Tube B: 500 µl Opti-MEM + 40 µl Lipo3000. The mixture in tube B was slowly added dropwise to tube A, gently mixed with a pipette, and then allowed to stand at room temperature for 15-20 min.

The transfection mix was added to the HEK-293T cells. After 4 h of transfection, the supernatant was discarded, and 10 ml of DMEM complete medium was added to each dish. The cells were then placed in a 37 °C, 5% CO₂ incubator for continued culture. After 48 h, the culture supernatant of the transfected 293T cells was collected, centrifuged at 400g for 5 min, and filtered through a 0.45 µm filter. The obtained filtrate was the stock recombinant lentivirus solution. The lentivirus was concentrated using an Utra-15 centrifugal filter device by centrifugation at 5000 rpm for 50 min. The concentrated lentivirus was aliquoted and stored at -80 °C for later use.

### 3. Lentivirus titer determination

HEK-293T cells were washed, digested, and counted. The concentrated lentivirus stock was serially diluted 10-fold (50×, 500×, 5000×, 50000×). A total of 2.5×10⁵ HEK-293T cells were added to the corresponding centrifuge tubes, each tube containing 500 µl of medium-virus-cell mixture. After mixing, the mixtures were transferred to a 24-well plate and cultured in a 37 °C, 5% CO₂ incubator. After 24 h, the medium was replaced. After 48 h, the infected HEK-293T cells were collected and subjected to hypoxia treatment. The proportion of cells positive for transgene expression was detected by flow cytometry, and the viral titer was calculated. Lentivirus titer was calculated as follows: Viral titer = (m × 2.5×10⁵ × dilution factor) / volume of transfection, where m is the proportion of transgene-positive cells.

### 4. Preparation of TILs from lung cancer

Lung cancer tumor pieces were placed in a 100 mm dish and washed with PBS. Necrotic areas and connective tissue were removed from the tumor pieces using sterile ophthalmic scissors or a scalpel, and the pieces were then cut into 1-3 mm³ fragments. The lung cancer tumor fragments were cultured in RPMI1640 medium supplemented with 10% AB serum, 1% glutamine, 1% bispecific antibody, and 6000 IU/mL IL-2. If no obvious adherent cells were observed under the microscope, the medium was changed the next day. If lymphocytes did not gradually increase or cancer cells did not decrease, the medium change was continued. If lymphocyte density significantly increased, e.g., cell clusters appeared, the culture was expanded and then placed back in a 37°C, 5% CO₂ incubator for continued culture. The culture was continued for no more than 10 days, after which TILs were collected. The collected cell suspension was filtered through a 40 µm filter to remove tumor fragments, centrifuged at 1000 rpm for 5 min, and resuspended in an appropriate amount of REP complete medium for later use.

### 5. Lentiviral infection of TILs

Based on the lentivirus titer, TILs were infected at an MOI of 10. Lentivirus (MOI=10), Lentiboost (100×), and TILs (3.5×10⁵) were added to REP complete medium. Each centrifuge tube contained 200 µl of medium-virus-cell mixture. After mixing, the mixture was transferred to a 48-well plate and cultured in a 37 °C, 5% CO₂ incubator. After 24 h of infection, the cells were collected, centrifuged at 1000 rpm, and the virus-containing supernatant was discarded. The cells were resuspended in an appropriate amount of REP complete medium, transferred to a 24-well plate, and cultured in a 37 °C, 5% CO₂ incubator.

### 6. Hypoxia treatment of lentivirus-infected TILs

After 72 h of culture, the lentivirus-infected TILs from step 5 were collected and counted. A total of 1×10⁶ TILs were transferred to a new 24-well plate and then placed in a hypoxic chamber (1% O₂) for hypoxia treatment (experimental group). The remaining TILs (non-hypoxic control group) were transferred to a new 24-well plate and cultured in a 37 °C, 5% CO₂ incubator. After 24 h of hypoxia, cells were counted, and 5×10⁵ cells from the experimental group and the control group were taken for flow cytometry detection.

### 7. Flow cytometry detection

The cell suspension from step 6 was counted, and 5×10⁵ cells were taken, centrifuged at 400g for 5 min, and the supernatant was discarded. The cells were resuspended in 100 µl PBS containing 2% FBS, and then 2.5 µl of Fc block (Fc receptor blocker) was added, followed by incubation at room temperature for 10 min. The cells were divided into two portions: a fully negative group and a test group, each with a volume of 50 µl (2.5×10⁵ cells). A mixed antibody solution containing IL-15 (Beijing BaiXinyi Biotechnology Co., A09D21-9E) and cetuximab injection (Merck) was added to the test group, followed by incubation at room temperature for 15 min. After incubation, 1 ml PBS containing 2% FBS was added, and the cells were centrifuged at 400 g for 5 min. The supernatant was discarded, and 50 µl of a mixed antibody solution containing FITC goat anti-human IgG Fcγ antibody (BioLegend, 398006) and Alexa Fluor 647 donkey anti-rabbit IgG antibody (BioLegend, 4064), prepared in PBS containing 2% FBS, was added to the test group, followed by incubation at room temperature for 10 min. Then, 50 µl of 7-AAD solution prepared in PBS containing 2% FBS was added to the test group, followed by incubation at room temperature for 7 min. After adding 1 ml PBS containing 2% FBS to both the fully negative group and the test group, the cells were centrifuged at 400g for 5 min. The supernatant was discarded, and the cells were resuspended in 200 µl PBS containing 2% FBS for analysis on a flow cytometer.

### 8. Results

After flow cytometry staining, the TIL cell suspension infected with the lentivirus containing the gene of interest was analyzed using a Beckman flow cytometer. The results are shown in Figure 1, panel B. Plasmid construction and lentiviral infection were successfully performed, yielding 001-TIL, 002-TIL, 003-TIL, and 004-TIL, respectively. Compared with uninfected TILs, the proportion of mIL-15+ cells in lentivirus-infected TILs was lower in the non-hypoxic group. After hypoxia treatment, the proportion of mIL-15+ cells in TILs were increased. Meanwhile, for lentivirus-infected TILs, compared with the non-hypoxic group, the proportion of hEGFRt+ cells did not change significantly after hypoxia treatment, as hEGFRt was regulated by the constitutive promoter SFFV.

These results showed that 3×HRE, 6×HRE, and 9×HRE were all able to induce mIL-15 expression. Unexpectedly, among the three promoters, the 3×HRE promoter produced the highest proportion of mIL-15+ cells under hypoxic induction conditions.

### Example 2: Detection of IL-15 in the culture supernatant of lentivirus-infected TILs

After 72 h of lentiviral infection, TILs were subjected to hypoxia treatment. After 24 h of hypoxia, the culture supernatants of non-hypoxic and hypoxic cells were collected. 100 µl of diluted standard and samples were added, and 100 µl of 1× dilution buffer R was added as a control well. The plate was sealed and incubated at room temperature (18-25 °C) for 2 h. Washing: The liquid in the wells was removed, 300 µl of 1× wash buffer working solution was added, left for 1 min, and then discarded. The washing step was repeated 3 times, and the plate was patted dry on filter paper each time. Then, 100 µl of biotinylated detection antibody human IL-15 working solution (Dakewe, 1111502) was added. The plate was sealed and incubated at room temperature (18-25 °C) for 1 h, followed by washing 3 times. Then, 100 µl of streptavidin-HRP working solution was added, the plate was sealed and incubated at room temperature (18-25 °C) for 30 min, followed by washing 3 times. Then, 100 µl of TMB was added, and the plate was incubated at room temperature (18-25 °C) in the dark for 5-30 min. Finally, 100 µl of stop solution was quickly added to terminate the reaction.

After 72 h of lentiviral infection with the gene of interest, lung cancer TILs were subjected to hypoxia treatment, and the culture supernatants of non-hypoxic and hypoxic cells were collected. IL-15 shedding was detected by ELISA.

The results are shown in Figure 2. Compared with the control group and the non-hypoxic group, no significant difference in IL-15 secretion was observed after hypoxia treatment, indicating that the membrane-bound IL-15 of the present invention did not undergo shedding.

### Example 3: mIL-15 promotes T_{SCM} cell formation and reduces T cell exhaustion

TILs infected with lentivirus carrying different plasmid constructs are named 001-TIL, 002-TIL, 003-TIL, and 004-TIL, respectively, were co-cultured with feeder cells K562-mIL21-4-1BBL for 7, 14, and 18 days. After co-culture, cell counting was performed, and T_{SCM} and T cell exhaustion were detected.

### 1. Construction of feeder cells K562-mIL21-4-1BBL

Construction of a plasmid containing the IL21 and 4-1BBL genes of interest: whole-gene synthesis (GenScript) was performed for the sequences of IL21, 4-1BBL, IRES, mCherry, and Puro, which were then cloned into the pLV-EF1a-c-MYC-RES-EGFP vector, replacing the original c-MYC-IRES-EGFP sequence. After obtaining the plasmid, lentiviral packaging was carried out, and the titer was calculated. K562 cells were infected with the lentivirus at an MOI of 10 based on the lentivirus titer. The lentivirus-infected K562 cells were treated with puromycin (2 µg/ml) to obtain the feeder cells K562-mIL21-4-1BBL. The expression of IL21 on K562-mIL21-4-1BBL cells was detected by flow cytometry.

The amino acid sequence of mIL21-4-1BBL is shown below:

MEYASDASLDPEAPWPPAPRARACRVLPWALVAGLLLLLLLAAACAVFLACPWAVS
GARASPGSAASPRLREGPELSPDDPAGLLDLRQGMFAQLVAQNVLLIDGPLSWYSDPGL
AGVSLTGGLSYKEDTKELVVAKAGVYYVFFQLELRRVVAGEGSGSVSLALHLQPLRSAA
GAAALALTVDLPPASSEARNSAFGFQGRLLHLSAGQRLGVHLHTEARARHAWQLTQGA
TVLGLFRVTPEIPAGLPSPRSEGACRAEGRGSLLTCGDVEENPGPHMMRSSPGNMERIVIC
LMVIFLGTLVHKSSSQGQDRHMIRMRQLIDIVDQLKNYVNDLVPEFLPAPEDVETNCEW
SAFSCFQKAQLKSANTGNNERIINVSIKKLKRKPPSTNAGRRQKHRLTCPSCDSYEKKPP
KEFLERFKSLLQKMIHQHLSSRTHGSEDSAAAFVPVFLPAKPTTTPAPRPPTPAPTIASQPL

SLRPEACRPAAGGAVHTRGLDFACDIYIWAPLAGTCGVLLLSLVIT (SEQ ID NO: 20).

### 2. Co-culture of lentivirus-infected TILs with K562-mIL21-4-1BBL

Puromycin-treated K562-mIL21-4-1BBL cells were collected and centrifuged at 1000 rpm for 10 min. After centrifugation, the supernatant was discarded, and the cells were washed three times with PBS. The cells were counted, and the cell density was adjusted to 1×10⁶/ml using RPMI1640 complete medium. Then, 6 µg/ml of mitomycin C was added, and after mixing, the cells were transferred to a T75 culture flask and incubated in a 37 °C, 5% CO₂ incubator for 18-24 h.

Day 1: After 72 h of lentiviral infection, TILs were subjected to hypoxia treatment. After 24 h of hypoxia, cells from the non-hypoxic and hypoxic groups were collected, centrifuged at 1000 rpm for 5 min, and the supernatant was discarded. TILs that were not infected with lentivirus were cultured in REP complete medium (containing IL-2 and anti-CD3), while lentivirus-infected TILs were cultured in REP complete medium (without IL-2 but containing anti-CD3). The cells were resuspended, mixed, and counted. Mitomycin C-treated K562-mIL21-4-1BBL cells were collected and centrifuged at 1000 rpm for 10 min. After centrifugation, the supernatant was discarded, and the cells were washed three times with PBS. K562-mIL21-4-1BBL cells were resuspended in REP complete medium and counted. TILs were co-cultured in a 24-well plates with K562-mIL21-4-1BBL at a ratio of 1:25. The cells were incubated in a 37 °C, 5% CO₂ incubator. The medium was replaced every 3 days (anti-CD3 was not added in subsequent medium changes). When cell confluence reached 80%, the cells were passaged.

Days 7 and 14: Co-cultured cells were collected and centrifuged at 1000 rpm for 5 min. The supernatant was discarded, and the cells were resuspended in the corresponding REP complete medium for cell counting.

Day 18: TILs were collected, counted, and subjected to hypoxia treatment for 24 h. After centrifugation at 1000 rpm for 5 min, the supernatant was discarded, and the cells were resuspended in the corresponding REP complete medium for cell counting. Then, 1×10⁶ cells were taken for flow cytometry analysis.

### 3. Flow cytometry detection

### (1) Flow cytometry detection of feeder cells K562-mIL21-4-1BBL:

Puromycin-treated lentivirus-infected K562 cells were collected and counted. A total of 5×10⁵ cells were taken, centrifuged at 400g for 5 min, and the supernatant was discarded. The cells were resuspended in 100 µl PBS containing 2% FBS, and 2.5 µl of Fc blocker was added, followed by incubation at room temperature for 10 min. The cells were divided into two portions: a fully negative group and a test group, each with a volume of 50 µl (2.5×10⁵ cells). An antibody mix containing Alexa Fluor 647 mouse anti-human IL-21 (BD Biosciences, 562043) was added (1.25 µl antibody per sample) to the test group, followed by incubation at room temperature for 8 min. After incubation, 50 µl of 7-AAD solution prepared in PBS containing 2% FBS was added to the test group, followed by incubation at room temperature for 7 min. Then, 1 ml of PBS containing 2% FBS was added to both the fully negative group and the test group, followed by centrifugation at 400g for 5 min. The supernatant was discarded, and the cells were resuspended in 200 µl PBS containing 2% FBS for analysis on a flow cytometer.

### (2) Flow cytometry detection of TILs:

After 18 days of co-culture with feeder cells, TILs were collected and counted. A total of 7.5×10⁵ cells were taken, centrifuged at 400g for 5 min, and the supernatant was discarded. The cells were resuspended in 150 µl PBS containing 2% FBS, and then 4 µl of Fc blocker was added, followed by incubation at room temperature for 10 min. The cells were divided into three portions: a fully negative group, Test Group 1, and Test Group 2, each with a volume of 50 µl (2.5×10⁵ cells). To Test Group 1, an antibody mix containing Alexa Fluor^{®} 700 anti-human CD45RA antibody (BioLegend, 304120), APC anti-human CD62L antibody (BioLegend, 304810), Brilliant Violet 510^{™} anti-human CD3 antibody (BioLegend, 317332), and APC/Cyanine7 anti-human CD8 antibody (BioLegend, 344714) was added (1.25 µl antibody per sample), followed by incubation at room temperature for 8 min. To Test Group 2, an antibody mix containing FITC anti-human CD127 (IL-7Rα) antibody (BioLegend, 351312), PE anti-human CD279 (PD-1) antibody (BioLegend, 621608), APC anti-human CD366 (Tim-3) antibody (BioLegend, 345012), Brilliant Violet 510^{™} anti-human CD3 antibody (BioLegend, 317332), and APC/Cyanine7 anti-human CD8 antibody (BioLegend, 344714) was added (1.25 µl antibody per sample), followed by incubation at room temperature for 8 min. After incubation, 50 µl of 7-AAD solution prepared in PBS containing 2% FBS was added to Test Group 1 and Test Group 2, followed by incubation at room temperature for 7 min. Then, 1 ml of PBS containing 2% FBS was added to the fully negative group and the test groups, followed by centrifugation at 400g for 5 min. The supernatant was discarded, and the cells were resuspended in 200 µl PBS containing 2% FBS for analysis on a flow cytometer.

### 4. Results

As shown in Figure 3, panel A, a schematic diagram of the feeder cell K562-mIL21-4-1BBL is presented. As shown in Figure 3, panel B, compared with K562 cells, the proportion of mIL21+ cells in feeder cells K562-mIL21-4-1BBL was 84.3%, indicating that the feeder cells K562-mIL21-4-1BBL were successfully constructed. As shown in Figure 3, panel C, compared with the control TILs cultured with IL-2 addition, the proliferation and viability of mIL-15-overexpressing TILs cultured without IL-2 addition showed no significant difference, indicating that mIL-15 overexpression in TILs promoted their proliferation. Among them, after hypoxia treatment, the proliferation of 002-TIL was superior to that of the non-hypoxic group.

As shown in Figure 3, panel D, under culture conditions without IL-2 addition, compared with the control TILs cultured with IL-2 addition, 001-TIL and 002-TIL overexpressing mIL-15 exhibited a significantly increased proportion of T_{SCM} cells. Compared with the non-hypoxic group, the proportion of T_{SCM} cells in 001-TIL and 002-TIL were increased after hypoxia treatment. Compared with 001-TIL (CD80-TM and CD80-Cyto), construct 002-TIL of the present inventionusing CD86-TM and CD86-Cyto showed a significantly higher proportion of T_{SCM}.

As shown in Figure 3, panel E, compared with control TILs cultured with IL-2 addition, the proportion of IL7Rα+ cells in mIL-15-overexpressing 002-TILs were increased. The increased proportions of T_{SCM} and IL7Rα⁺ cells in 002-TIL indicated that mIL-15 overexpression enhanced stemness. Meanwhile, as shown in Figure 3, panel F, under culture conditions without IL-2 addition, compared with control TILs cultured with IL-2 addition, 001-TIL and 002-TIL overexpressing mIL-15 showed significantly reduced expression of PD-1 and Tim-3 in CD8+ T cells. Compared with 001-TIL (CD80-TM and CD80-Cyto), construct 002-TIL of the present invention using CD86-TM and CD86-Cyto showed significantly lower expression of PD-1 and Tim-3 in CD8+ T cells.

These results demonstrated that mIL-15 overexpression in TILs promoted the generation of T_{SCM} cells and reduced the expression of exhaustion markers. Moreover, the effect of CD86-TM-CD86-Cyto was significantly superior to that of CD80-TM-CD80-Cyto.

### Example 4: TIL-mIL15 cells exhibited a less differentiated phenotype

To further confirm the effects of mIL15 on T cell differentiation, exhaustion, and apoptosis, UN-TIL+IL2, TIL-mIL15+IL2, and TIL-mIL15-IL2 cells expanded for 14 days using the K562-mIL21-4-1BBL culture platform was subjected to transcriptome sequencing and flow cytometry analysis.

### 1. RNA-Seq analysis

UN-TIL+IL2, TIL-mIL15+IL2, and TIL-mIL15-IL2 cells cultured on the K562-mIL21-4-1BBL cell platform were collected, and 5×10⁶ cells were used for transcriptome sequencing to analyze differences in gene expression. Total RNA was extracted using the EASYspinPlus Tissue/Cell RNA Rapid Extraction Kit (Beijing Aidlab, #RN2802). A cDNA library was constructed using Illumina's NEBNext^{®} UltraTM RNA Library Prep Kit. The insert size of the cDNA library was examined using an Agilent 2100 bioanalyzer. After meeting expectations, the effective concentration of the cDNA library was accurately quantified by qRT-PCR, followed by Illumina sequencing. For data analysis, Reads Per Kilobase per Million mapped reads (RPKM) were calculated. Differentially expressed genes were identified with log2(fold change) > 1.5 or log2(fold change) < -1.5, and statistical significance (p < 0.05) was determined using edgeR.

### 2. Flow cytometry detection of TCF1

(1) Surface staining: After 14 days of co-culture of TILs with K562-mIL21-4-1BBL cells, the cells were collected and counted. A total of 2×10⁶ cells were taken and stained with Brilliant Violet 510^{™} anti-human CD3 antibody (BioLegend, 317332) and eFluor 600 EGFR antibody (eBioscience, 50-9509-42).
(2) TCF1 staining: After surface staining, the cells were subjected to Zombie staining and washed with 1×PBS. After fixation using the True-Nuclear Transcription Factor Buffer Set (BioLegend, #424401), the cells were stained with PE-anti-TCF1 antibody (BioLegend, 655208).

### 3. Results

As shown in Figure 4, panel A, RNA-seq analysis revealed that, compared with UN-TIL+IL2 cells, 1344 up-regulated genes and 51 down-regulated genes were identified in TIL-mIL15+IL2 cells, while 1858 up-regulated genes and 160 down-regulated genes were identified in TIL-mIL15-IL2 cells. Compared with TIL-mIL15+IL2 cells, 164 up-regulated genes and 239 down-regulated genes were identified in TIL-mIL15-IL2 cells. As shown in Figure 4, panel B, enrichment of multiple signaling pathways was also observed in TIL-mIL15-IL2 cells, among which the JAK-STAT, mTOR, and PI3K-Akt signaling pathways were closely related to the regulation of mIL15. As shown in Figure 4, panel C, gene signatures related to T cell differentiation and memory were enriched in TIL-mIL15-IL2 cells. As shown in Figure 4, panel D, the expression of genes related to T cell differentiation and effector function, including *PRDM1* (encoding BLIMP-1), *ID2, EOMES, IFNG, GZMB*, and *TNF*, was significantly decreased in TIL-mIL15-IL2 cells, while the expression of *TCF7* (encoding TCF1), a marker of memory stem-like T cells, was significantly increased. Moreover, the expression of the survival genes *BCL2* and *BCL2L1* was significantly increased. In addition, the expression of genes associated with inhibitory regulators, including *LAG3, HAVCR2* (encoding TIM3), *TIGIT, TOX,* and *CTLA4*, was decreased in TIL-mIL15-IL2 cells. As shown in Figure 4, panel E, the expression level of TCF1 in TIL-mIL15-IL2 cells was much higher than that in UN-TIL+IL2 and TIL-mIL15+IL2 cells.

### Example 5: Anti-tumor activity of mIL-15-overexpressing TILs

To verify whether mIL-15 overexpression in TILs promotes their anti-tumor activity and to determine the differences between CD80-TM+CD80-Cyto and CD86-TM+CD86-Cyto in the functional performance of mIL-15, 001-TIL and 002-TIL cells were co-cultured with Calu6 tumor cells. Tumor cell viability and IFN-γ secretion were detected using CCK8 and ELISA, respectively. The experimental methods were as follows:

### 1. Detection of tumor cell viability by CCK8 assay

A total of 1×10⁵ Calu6 cells were seeded into a 24-well plate. After 72 h of lentiviral infection, TILs were subjected to hypoxia treatment. After 24 h of hypoxia, TILs from the non-hypoxic and hypoxic groups were collected and centrifuged at 1000 rpm for 5 min. The cells were resuspended in REP complete medium, and the cell density was adjusted to 1.5×10⁶/ml. The culture supernatant of Calu6 cells in the 24-well plate was discarded, and 1 ml of the non-hypoxic or hypoxic TIL cell suspension (i.e., 1.5×10⁶ TILs) was added to each well. After 24 h of incubation in a 37°C, 5% CO₂ incubator, the cell culture supernatant was collected. The cells were washed once with PBS, and tumor cell viability was detected using the CCK8 assay.

### 2. Detection of IFN-γ secretion by ELISA

The collected cell culture supernatants were centrifuged at 400g for 5 min, and the supernatants were transferred to a new centrifuge tube. The required number of strip wells was determined based on the number of experimental wells (blank and standard). Then, 100 µl of diluted standard and samples were added, and 100 µl of 1× dilution buffer R was added as a control well. Subsequently, 100 µl of biotinylated anti-human IFN-γ antibody (Dakewe, 1110002) working solution was added. The plate was sealed and incubated at room temperature (18-25°C) for 1 h. Washing: The liquid in the wells was removed, 300 µl of 1× wash buffer working solution was added, left for 1 min, and then discarded. The washing step was repeated three times, and the plate was patted dry on filter paper each time. Then, 100 µl of streptavidin-HRP working solution was added, the plate was sealed and incubated at room temperature (18-25°C) for 20 min, followed by three washes. Color development: 100 µl of TMB was added, and the plate was incubated at room temperature (18-25°C) in the dark for 5-30 min. Termination: 100 µl of stop solution was quickly added to terminate the reaction.

### 3. Results

The results are shown in Figure 5, Table 1, and Table 2.

As shown in Figure 5, panel A, compared with Calu6 tumor cells cultured alone, the viability of Calu6 tumor cells co-cultured with 001-TIL or 002-TIL was significantly reduced. Moreover, compared with 001-TIL cells, 002-TIL cells exhibited stronger anti-tumor activity.

As shown in Figure 5, panel B, compared with mIL-15-overexpressing TILs cultured alone, the secretion of IFN-γ in the culture supernatant was significantly increased when 001-TIL or 002-TIL cells were co-cultured with Calu6 tumor cells. Furthermore, compared with 001-TIL cells, 002-TIL cells showed higher IFN-γ secretion.

**Table 1. Anti-tumor activity of different TILs**

| Tumor cell viability (%) | Calu6 | TILs+Calu6 |
|---|---|---|
| Control TIL | 100 | 67.79 |
| 001-TIL | 100 | 40.79 |
| 002-TIL | 100 | 12.875 |

**Table 2. IFN-γ secretion of different TILs**

| IFN-γ secretion (pg/mL) | TILs | TILs+Calu6 |
|---|---|---|
| Control TIL | 94.72 | 141.87 |
| 001-TIL | 62.30 | 127.82 |
| 002-TIL | 195.03 | 323.91 |

The above experimental results demonstrated that mIL-15 overexpression in TILs promoted their anti-tumor activity. Moreover, the anti-tumor activity and IFN-γ secretion of 002-TIL were significantly superior to those of 001-TIL, indicating that the use of CD86-TM and CD86-Cyto was significantly more effective than the CD80-TM and CD80-Cyto construct.

### Example 6: Cytotoxic effects

In a clinical setting, to further improve the safety of TIL cell therapy overexpressing mIL-15, the present invention provides a membrane surface domain (hEGFRt) as an immune brake element and/or recognition element. The hEGFRt-specific monoclonal antibody cetuximab can mediate complement-dependent cytotoxicity (CDC) and NK cell-mediated cytotoxicity (ADCC) to effectively lyse TILs expressing mIL-15.

CDC is a process in which the Fc portion of an antibody binds to and activates complement molecules, while the Fab portion of the antibody binds to target cells, leading to the formation of a membrane attack complex and ultimately target cell lysis. In the present invention, hEGFRt expressed on TILs binds to cetuximab and then to the added complement molecules, resulting in lysis of TIL.

ADCC refers to the binding of the Fab segment of an antibody to the epitope of lentivirus-infected cells or tumor cells, while its Fc segment binds to Fc receptors on the surface of killer cells (NK cells, macrophages, neutrophils, etc.), mediating direct killing of target cells by killer cells. It is an important mechanism by which anti-tumor therapeutic antibody drugs exert their effects.

The experimental methods were as follows:

### (1) Complement-dependent cytotoxicity (CDC) assay

Lentivirus-infected TILs were co-cultured with K562-mIL21-4-1BBL cells for 18 days and then subjected to hypoxia treatment. The cells were collected and centrifuged at 1000 rpm for 5 min. The supernatant was discarded, and the cells were resuspended in the corresponding REP complete medium for cell counting. A total of 5×10⁵ cells were taken for the CDC assay. Using a 48-well plate with 200 µl of the culture system, three experiment groups were set: TILs alone, TILs + 100 µg/ml cetuximab, and TILs + 25% complement + 100 µg/ml cetuximab. After 4 h of incubation in a 37 °C, 5% CO₂ incubator, the cells were collected for flow cytometry analysis.

### (2) Antibody-dependent cellular cytotoxicity (ADCC) assay

Lentivirus-infected TILs were co-cultured with K562-mIL21-4-1BBL cells for 18 days and then subjected to hypoxia treatment. The cells were collected and centrifuged at 1000 rpm for 5 min. The supernatant was discarded, and the cells were resuspended in the corresponding REP complete medium for cell counting. A total of 1×10⁶ cells were taken for the ADCC assay. Using a 24-well plate with 1 ml of the culture system. The experiment was divided into four groups: TILs alone, TILs + 100 µg/ml cetuximab, TILs + NK cells (effector-to-target ratio 1:10) + 100 µg/ml cetuximab, and TILs + NK cells (effector-to-target ratio 1:20) + 100 µg/ml cetuximab. After 24 h of incubation in a 37 °C, 5% CO₂ incubator, the cells were collected for flow cytometry analysis.

The experimental results are shown in Figure 6. Compared with 002-TIL cells alone and 002-TIL cells with cetuximab, the proportion of mIL-15+ TILs were significantly reduced in the 002-TIL cells treated with complement and cetuximab for 4 h (Figure 6, panel A). Compared with 002-TIL cells alone and 002-TIL cells with cetuximab, the proportion of mIL-15+ TILs was significantly reduced in the 002-TIL cells treated with NK cells (1:10 and 1:20) and cetuximab for 24 h (Figure 6, panel B).

These results indicated that the molecular brake element based on the mechanism of action of the hEGFRt extracellular domain had high safety and efficacy.

### Example 7: Different linking methods of the immune brake element hEGFRt

In addition to using the constitutive promoter SFFV for linking, the immune brake element hEGFRt in the present invention was also linked using a polypeptide linker sequence T2A that can be cleaved *in vivo*. The DNA expression cassette of the fusion protein formed by linking hEGFRt via T2A was translated into a fusion polypeptide *in vivo*, which was simultaneously cleaved *in vivo* upon translation. hEGFRt was transported to the extracellular space via a signal peptide and anchored to the cell membrane via its transmembrane region in C-terminal. As shown in Figure 7, panel A, construct 005 was generated, in which the immune brake element was linked via the polypeptide linker T2A. Using molecular cloning techniques, the SFFV promoter sequence in construct 002 was replaced with the T2A sequence.

As shown in Figure 7, panel B, after lentiviral packaging and infection of TILs for 5 days followed by hypoxia treatment for 24 h, the proportions of mIL-15 and hEGFRt were detected by flow cytometry. The results showed that plasmid construction and lentiviral infection were successful. Compared with uninfected TILs, the proportion of mIL-15+ cells in TILs infected with the 005 lentivirus was lower without hypoxia treatment. After hypoxia treatment, the proportion of mIL-15+ cells in TILs was increased.

As shown in Figure 7, panel C, compared with uninfected TILs, the gMFI of mIL-15 in TILs infected with the 005 lentivirus was lower without hypoxia treatment. After hypoxia treatment, the gMFI of mIL-15 in TILs was significantly increased. Meanwhile, compared with the non-hypoxic group, the gMFI of hEGFRt in lentivirus-infected TILs was significantly increased after hypoxia treatment. As shown in Figure 7, panel D, under IL-2-addition conditions, both uninfected TILs and 005 lentivirus-infected TILs proliferated continuously until day 13, with cell viability ≥90%. Under IL-2-free conditions, compared with uninfected TILs, 005 lentivirus-infected TILs proliferated continuously until day 13, with cell viability ≥90%.

These results showed that the immune brake element hEGFRt linked via T2A could also be induced to express. Under IL-2-free conditions, TILs infected with the 005 lentivirus proliferated continuously, with cell viability ≥90%. The results indicated that when linked via T2A, the immune brake element hEGFRt could still be induced to express. *In vitro*, mIL-15 overexpression promoted TIL cell proliferation, avoiding the clinical side effects associated with the use of IL-2.

### Example 8: Lentiviral infection of cervical cancer TILs

In this example, it was further investigated whether expression of mIL-15 of the present invention can promote stemness or reduce exhaustion marker expression in cervical cancer TILs. The methods were as follows:
Preparation of cervical cancer TILs: Cervical cancer tumor pieces were placed in a 100 mm dish and washed once with PBS. Necrotic areas and connective tissue were removed from the tumor pieces using sterile ophthalmic scissors or a scalpel, and the pieces were then cut into 1-3 mm³ fragments. The cervical tumor fragments were cultured in RPMI1640 medium supplemented with 10% AB serum, 1% glutamine, 1% bispecific antibody, and 6000 IU/mL IL-2. If no obvious adherent cells were observed under the microscope, the medium was changed the next day. If lymphocytes did not gradually increase or cancer cells did not decrease, the medium change was continued. If lymphocyte density significantly increased, e.g., cell clusters appeared, the culture was expanded and then placed back in a 37 °C, 5% CO₂ incubator for continued culture. The culture was continued for no more than 10 days, after which the cells were collected. The collected cell suspension was filtered through a 40 µm filter to remove tumor fragments, centrifuged at 1000 rpm for 5 min, and resuspended in an appropriate amount of REP complete medium for later use.

Using the method described in Example 1, the 005 construct was introduced into the cervical cancer TILs, and the stemness of TILs as well as the expression of exhaustion markers were observed.

As shown in Figure 8, panel A, after cervical cancer TILs were infected with the 005 lentivirus for 5 days and then subjected to hypoxia treatment for 24 h, mIL-15 and hEGFRt were detected by flow cytometry. The results showed that compared with uninfected TILs, the proportions of mIL-15+ and hEGFRt+ cells in 005 lentivirus-infected TILs were lower without hypoxia treatment. After hypoxia treatment, the proportions of mIL-15+ and hEGFRt+ cells in TILs were increased.

As shown in Figure 8, panel B, compared with uninfected TILs, the gMFI of mIL-15 and hEGFRt in 005 lentivirus-infected TILs was increased after hypoxia treatment.

As shown in Figure 8, panel C, under both non-hypoxic and hypoxic conditions, compared with control TILs cultured with IL-2 addition, 005-TILs cultured without IL-2 addition overexpressed mIL-15, and the proportion of T_{SCM} cells was significantly increased.

As shown in Figure 8, panel D, under both non-hypoxic and hypoxic conditions, compared with control TILs cultured with IL-2 addition, the proportion of IL7Rα+ cells in mIL-15-overexpressing 005-TILs cultured without IL-2 addition was significantly increased. In cervical cancer patient TILs overexpressing mIL-15, the proportions of T_{SCM} and IL7Rα+ cells were increased, indicating that mIL-15 enhanced their stemness.

Meanwhile, as shown in Figure 8, panel E, under both non-hypoxic and hypoxic conditions, compared with control TILs cultured with IL-2 addition, 005-TILs cultured without IL-2 addition overexpressed mIL-15, and the expression of TIGIT and Tim-3 in CD8+ T cells was significantly reduced.

These results showed that expression of mIL-15 of the present invention in cervical cancer TILs unexpectedly and significantly promoted the generation of T_{SCM} cells and significantly reduced the expression of exhaustion markers.

### Discussion

Long-term immune surveillance by persistently existing TILs may be the key to achieving durable responses in adoptive cell therapy (ACT). In mouse and non-human primate models, adoptively transferred effector CD8+ T cells derived from central memory (T_{CM}) or naïve (T_{N}) T cell subsets have shown greater therapeutic potential. Therefore, T cell subsets corresponding to a less differentiated state are of great interest because of their potential to provide superior clinical utility. T memory stem cells (T_{SCM}) are the least differentiated memory T cell subset identified to date, so the preparation of T_{SCM} suitable for cell therapy is crucial.

IL-15 is a pro-survival cytokine required for the maintenance of long-lived CD8+ memory T cell homeostasis. It inhibits activation-induced cell death (AICD), enhances *in vivo* anti-tumor activity, and reverses T cell anergy. Unlike IL-2, IL-15 does not drive Treg expansion. Moreover, IL-15 is required for the generation of innate T cells involved in immune surveillance and impeding tumor growth. IL-15 is considered an immunotherapeutic agent for cancer treatment, acting on natural killer (NK) cells or T cells. Monomeric IL-15 is a small, unstable protein with a short serum half-life, requiring supraphysiological administration to achieve *in vivo* responses. Physiological presentation of IL-15 requires the IL-15 receptor α chain (IL-15Rα), and recombinant soluble IL-15 binds to recombinant soluble IL-15Rα to enhance the anti-tumor activity of T cells.

Based on the above research background, to improve the persistence and memory potential of infused T cells, patent application 202210316484.5 employed a Tet-on system, adding the inducer tetracycline to induce expression of IL-7 and/or IL-15. Although that method improved TIL cell expansion, the Tet-on system contains the C-terminus of the herpes simplex virus (HSV) VP16 protein, which has immunogenicity. Moreover, the system requires the inducer tetracycline or tetracycline derivatives to induce secretion and expression of IL-7 and IL-15, causing side effects related to the inducer. Patent application US2022/0133801A1 used acetazolamide to induce mIL-15 expression in TILs. This method avoided the toxicity of high-dose IL-2 and promoted the anti-tumor activity of TILs. However, this method introduced a drug, which has potential clinical side effects. At the same time, with this method, after the addition of the small-molecule drug, IL-15 could be expressed in multiple organs and tissues, lacking selectivity for enrichment in the tumor microenvironment, thus posing certain safety risks.

Hypoxia is a hallmark feature of solid tumors. Due to insufficient blood supply, oxygen concentrations can vary between 0.02% and 2% O₂, compared to 2% to 9% O₂ in normal tissues (Sharma A, Arambula JF, Koo S, Kumar R, Singh H, Sessler JL, et al. Hypoxia-targeted drug delivery. Chem Soc Rev. 2019;48(3):771-813.). Exploiting this unique environmental signal for targeted therapy is a strategy in cancer treatment. Therefore, by taking advantage of the hypoxic microenvironment of solid tumors, the present invention uses HREs to induce expression of membrane-bound IL-15 (mIL-15) in engineered TILs, thereby maintaining long-term persistence of a memory stem cell phenotype. The use of the CD86 transmembrane region and intracellular domain further increases the proportion of stem-like T cells in TILs and enhances their anti-tumor activity. Truncated hEGFRt is used in the molecular construct to further improve safety. mIL-15 provides an ideal signaling pathway that extends the persistence of TILs infused into patients, thereby providing durable immune surveillance and therapeutic potential.

All documents mentioned in the present invention are incorporated by reference herein as if each document were individually incorporated by reference. In addition, it should be understood that after reading the above teachings of the present invention, those skilled in the art may make various changes or modifications, and these equivalent forms shall also fall within the scope defined by the appended claims of the present invention.

## Claims

1. A fusion polypeptide comprising the following elements:
(i) interleukin 15 (IL-15) or a functionally active fragment thereof;
(ii) a transmembrane domain; and
(iii) an intracellular domain;
wherein, the intracellular domain is a CD86 intracellular domain.

2. The fusion polypeptide of claim 1, wherein the structural formula of the fusion polypeptide is as shown in formula (I):
X-IL15-L-H-TM-Cyto (I)
wherein,
"-" each independently is absent or a linker peptide;
X is absent or a signal peptide;
IL15 is the interleukin 15 element;
L is absent or a linker;
H is a hinge region;
TM is a transmembrane domain;
Cyto is an intracellular domain, wherein the intracellular domain is a CD86 intracellular domain.

3. The fusion polypeptide of claim 1, wherein the amino acid sequence of the CD86 intracellular domain is set forth in SEQ ID NO: 2.

4. The fusion polypeptide of claim 2, wherein the transmembrane domain is a CD86 transmembrane domain, and/ or the hinge region is a CD86 hinge region.

5. The fusion polypeptide of claim 1, wherein the sequence of the fusion protein is set forth in SEQ ID NO: 7.

6. A polynucleotide, which encodes the fusion polypeptide of claim 1.

7. A nucleic acid construct, which comprises a first expression cassette, wherein the first expression cassette comprises a hypoxia-responsive promoter and the polynucleotide of claim 6.

8. A vector, which comprises the polynucleotide of claim 6.

9. An immune cell, which comprises the fusion polypeptide of claim 1, the polynucleotide of claim 6, the nucleic acid construct of claim 7, and/or the vector of claim 8.

10. The cell of claim 9, wherein the cell is a tumor infiltrating lymphocyte (TIL).

11. A composition, which comprises the fusion polypeptide of claim 1, the polynucleotide of claim 6, the nucleic acid construct of claim 7, the vector of claim 8, and/or the cell of claim 9, and a pharmaceutically acceptable carrier.

12. A kit, which comprises the fusion polypeptide of claim 1, the polynucleotide of claim 6, the nucleic acid construct of claim 7, the vector of claim 8, the cell of claim 9, and/or the composition of claim 11.

13. Use of the fusion polypeptide of claim 1, the polynucleotide of claim 6, the nucleic acid construct of claim 7, the vector of claim 8, the cell of claim 9, the composition of claim 11, and/or the kit of claim 12 in the preparation of a medicament for preventing, alleviating and/or treating a tumor.

14. The use of claim 13, wherein the tumor is a solid tumor, a hematological tumor, or a combination thereof.

15. A method for treating a disease, which comprises administering the immune cell of claim 9 or the composition of claim 11 to a subject.
